(19) Europäisches Patentamt
European Patent Office
Office européen des brevets

(11) Veröffentlichungsnummer : **0 031 574**
**B1**

(12) **EUROPÄISCHE PATENTSCHRIFT**

(45) Veröffentlichungstag der Patentschrift : **15.12.82**

(51) Int. Cl.³ : **C 07 F 9/09**, C 07 F 9/165, C 07 F 9/24, C 07 F 9/40, A 01 N 57/08

(21) Anmeldenummer : **80108102.7**

(22) Anmeldetag : **22.12.80**

(54) **Oximinophosphorsäurederivate, Verfahren zu ihrer Herstellung und ihre Verwendung zur Bekämpfung von Schädlingen.**

(30) Priorität : **29.12.79 DE 2952738**

(43) Veröffentlichungstag der Anmeldung : **08.07.81 (Patentblatt 81/27)**

(45) Bekanntmachung des Hinweises auf die Patenterteilung : **15.12.82 Patentblatt 82/50**

(84) Benannte Vertragsstaaten : **BE CH DE FR GB IT LI NL**

(56) Entgegenhaltungen :
**DE A 2 261 189**
**DE A 2 304 848**
**DE B 1 052 981**
**DE B 1 238 902**

(73) Patentinhaber : **BASF Aktiengesellschaft**
**Carl-Bosch-Strasse 38**
**D-6700 Ludwigshafen (DE)**

(72) Erfinder : **Buerstinghaus, Rainer, Dr.**
**Weinbergstrasse 85**
**D-6940 Weinheim-Luetzelsachsen (DE)**
Erfinder : **Kiehs, Karl, Dr.**
**Sudetenstrasse 22**
**D-6840 Lampertheim (DE)**
Erfinder : **Siegel, Hardo, Dr.**
**Hans-Purrmann-Allee 25**
**D-6720 Speyer (DE)**
Erfinder : **Adolphi, Heinrich, Dr.**
**Kalmitweg 11**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 031 574 B1

# Oximinophosphorsäurederivate, Verfahren zu ihrer Herstellung und ihre Verwendung zur Bekämpfung von Schädlingen

Die vorliegende Erfindung betrifft Oximinophosphorsäurederivate, Verfahren zu ihrer Herstellung, Schädlingsbekämpfungsmittel, die diese Phosphorsäurederivate als Wirkstoffe enthalten, sowie ein Verfahren zur Bekämpfung von Schädlingen mit diesen Wirkstoffen.

Oximinophosphorsäurederivate sind aus der DE-AS 10 52 981, der DE-AS 12 38 902 und der DE-OS 23 04 848 bekannt. Sie eignen sich zur Bekämpfung von Insekten und Spinnentieren.

Es wurde gefunden, daß Oximinophosphorsäurederivate der Formel I

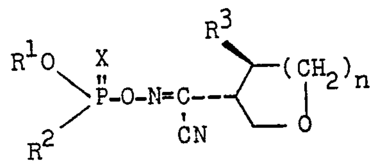

(I)

in der

$R^1$ eine unverzweigte oder verzweigte Alkylgruppe mit bis zu 4 Kohlenstoffatomen,

$R^2$ eine unverzweigte oder verzweigte Alkoxy- oder Alkylthiogruppe mit bis zu 4 Kohlenstoffatomen, eine unverzweigte oder verzweigte Alkylgruppe mit bis zu 3 Kohlenstoffatomen, Phenyl, die Aminogruppe oder einen unverzweigten Alkylamino- oder Dialkylaminorest mit jeweils bis zu 4 Kohlenstoffatomen in einer Alkylgruppe,

$R^3$ Wasserstoff oder Methyl,

X Sauerstoff oder Schwefel und

n 1 oder 2 bedeuten,

insektizid, akarizid und nematizid sehr gut wirksam und bekannten Wirkstoffen ähnlicher Struktur bzw. gleicher Wirkungsrichtung überlegen sind.

Unverzweigte oder verzweigte Alkylreste für $R^1$ sind beispielsweise Methyl, Ethyl, Propyl, Isopropyl, Butyl, Isobutyl, unverzweigte oder verzweigte Alkyl-, Alkoxy- oder Alkylthioreste für $R^2$ sind beispielsweise Methyl, Ethyl, n-Propyl, Isopropyl, Methoxy, Ethoxy, Propoxy- und Butoxyreste, Methylthio, Ethylthio, n-Propylthio, Isopropylthio, n-Butylthio, sec.-Butylthio, Isobutylthio. Als Alkylamino- bzw. Dialkylaminoreste kommen für $R^2$ beispielsweise Methylamino, Dimethylamino, Ethylamino, Diethylamino, Methylethylamino, n-Butylamino, Di-n-butylamino in Betracht.

Bevorzugte Substituenten für $R^1$ sind Methyl und Ethyl ; bevorzugte Substituenten für $R^2$ sind Methoxy, Ethoxy, Methyl, Ethyl, Phenyl, Amino, Methylamino, Dimethylamino und Isopropylamino.

Die Oximinophosphorsäurederivate der Formel I können durch Umsetzung von α-Oximinonitrilen der Formel II gegebenenfalls in Gegenwart eines Säureacceptors oder durch Umsetzung von Alkalimetall-, Erdalkalimetall- oder gegebenenfalls substituierten Ammoniumsalzen dieser α-Oximinonitrile mit (Thiono) (Thiol)Phosphor(Phosphon)säureester(amid) halogeniden der Formel III nach folgender Reaktionsgleichung erhalten werden :

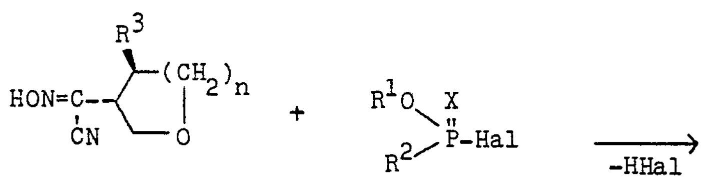


(II) (III)

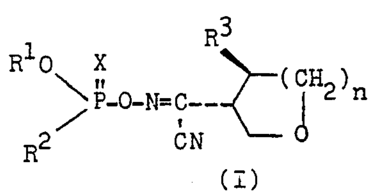

(I)

Dabei haben die Substituenten $R^1$, $R^2$, $R^3$ und n die oben angegebene Bedeutungen, und Hal steht für Halogen, vorzugsweise für Chlor.

Die Umsetzung wird zweckmäßigerweise in gegenüber den Reaktionsteilnehmern inerten Lösungs- oder Verdünnungsmitteln ausgeführt. Hierzu sind beispielsweise geeignet : aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlorethan, Chlorbenzol, wie Diethyl- und Di-n-butylether, Methyl-tert.-butylether, Tetrahydrofuran, Dioxan ; Ketone, beispielsweise Aceton, Methylethylketon, Methylisopropylketon ; ferner Nitrile, wie Acetonitril und Propionitril. Auch Gemische dieser Lösungs- oder Verdünnungsmittel können verwendet werden.

Als Säureacceptoren können die bei der Phosphorylierung von Hydroxyverbindungen üblichen basischen Mittel Verwendung finden. Als besonders geeignet erweisen sich Alkalimetallcarbonate oder -alkoholate, wie Natrium- und Kaliumcarbonat, -methylat und -ethylat, ferner aliphatische, aromatische und heterocyclische Amine, z.B. Triethylamin, Dimethylamin, Piperidin, Dimethylanilin, Dimethylbenzylamin und Pyridin. In einigen Fällen erweist sich die Verwendung von Alkyllithiumverbindungen, z.B. n-Butyllithium, als vorteilhaft.

Statt in Gegenwart eines Säureacceptors zu arbeiten, ist es ebenso gut möglich, zunächst die Salze, etwa die Alkalimetall-, Erdalkalimetall oder Ammoniumsalze der α-Oximinonitrile der Formel II in Substanz herzustellen und diese anschließend mit der Verbindung der Formel III weiter umzusetzen.

Üblicherweise setzt man die Ausgangsstoffe in äquimolaren Verhältnissen ein. Ein Überschuß der einen oder anderen Reaktionskomponente kann in einigen Fällen Vorteile bringen.

Die Reaktionstemperatur ist innerhalb eines größeren Bereiches variierbar. Im allgemeinen arbeitet man zwischen 0 und 120 °C, vorzugsweise im Bereich zwischen 20 und 50 °C. Da die Reaktion in einigen Fällen exotherm verläuft, kann eine Außenkühlung zu Beginn der Reaktion von Vorteil sein.

Die Umsetzung wird üblicherweise bei Normaldruck ausgeführt.

Die zur Herstellung der Verbindungen I als Ausgangsmaterialien verwendeten α-Oximinonitrile der Formel II sind nicht bekannt. Sie lassen sich aber in an sich bekannter Weise (DE-AS 15 67 142) durch Chlorieren der zugehörigen 3-Formyldihydropyran(furan)oxime der Formel IV und anschließende Umsetzung mit Natrium- oder Kaliumcyanid gemäß folgender Reaktionsgleichung herstellen :

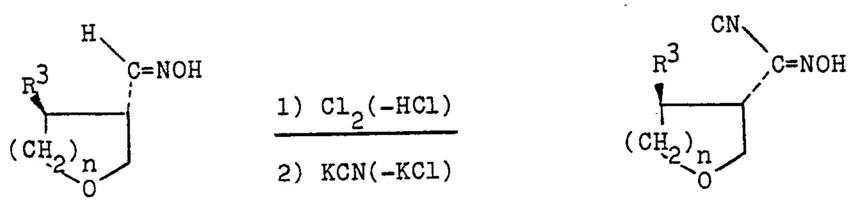


IV II

Oxime der Formel IV erhält man durch Umsetzung des betreffenden 3-Formyldihydropyrans(furans) der Formel V, das durch Hydroformylierung des entsprechenden Olefins in Gegenwart von Rhodiumkatalysatoren hergestellt werden kann, mit Hydroxylaminhydrochlorid nach folgender Reaktionsgleichung :

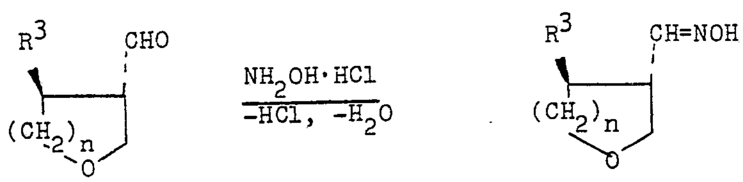


V IV

In diesen Reaktionsschemata hat $R^3$ die obengenannten Bedeutungen und n steht für 1 oder 2.

Die zur Synthese der Verbindungen der Formel I außerdem benötigten (Thiono) (Thiol)Phosphor(Phosphon)säureester(amid)halogenide III sind aus Houben-Weyl, Methoden der organischen Chemie, Band XII/2, S. 274 ff, Georg Thieme-Verlag, Stuttgart, 1964 bekannt und lassen sich auf den dort beschriebenen Synthesewegen herstellen.

Die neuen Verbindungen der Formel I fallen teilweise in Form farbloser oder schwach bräunlich gefärbter Öle an, die sich durch längeres Erwärmen unter vermindertem Druck auf mäßig erhöhte Temperatur (« Andestillieren ») von den letzten flüchtigen Anteilen befreien und auf diese Weise reinigen lassen. Erhält man die Verbindungen der Formel I in kristalliner Form, so kann ihre Reinigung durch Umkristallisation erfolgen.

Da die Oximinophosphorsäurederivate der Formel I in den strukturisomeren syn- und anti-Formen auftreten können, eignen sich ihre Schmelz- bzw. Siedebereiche wenig zu ihrer Charakterisierung ; zu diesem Zweck dienen daher im folgenden H-NMR-Spektren und Elementaranalyse.

Die folgenden Beispiele erläutern die Herstellung der Oximinophosphorsäurederivate der Formel I.

Beispiel 1

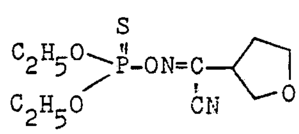

52,8 g fein gepulvertes Natriumhydroxid werden in einer Mischung aus 130 ml Wasser und 400 ml Ethanol gelöst. Unter Eiskühlung fügt man 91,6 g Hydroxylaminhydrochlorid in 95 ml Wasser hinzu, saugt vom ausgefallenen Natriumchlorid ab und tropft das Filtrat unter heftigem Rühren bei maximal 30 °C zu 120 g 3-Formyltetrahydrofuran (Kp : 103 °C/133 mbar). Die Reaktionsmischung wird anschließend 12 Stunden bei Raumtemperatur gerührt und in Wasser gegossen. Die wäßrige Phase wird mit Kochsalz gesättigt und ca. achtmal mit jeweils 125 ml Ether extrahiert. Die vereinigten Extrakte werden über Natriumsulfat getrocknet. Der nach dem Abziehen des Lösungsmittels verbleibende Rückstand wird bei 0,4 mbar destilliert, wobei zwischen 78 °C und 82 °C 88 g 3-Formyltetrahydrofuranoxim als farblose Flüssigkeit übergehen ; Ausbeute : 64 % der Theorie.

$C_5H_9NO_2$ (115)

Ber. : C 52,2 H 7,9 N 12,2

Gef. : C 52,4 H 8,1 N 12,3

60-MHz-H-NMR-Spektrum in $CDCl_3$(δ-Werte) :

1,8-2,65 (2H), 2,8-3,6 (1H), 3,5-4,4 (4H), 6,9 (syn), 7,6 (anti) (1H), 9,1-10,2 (1H).

In eine gut gekühlte Lösung von 128 g 3-Formyltetrahydrofuranoxim in 1 400 ml Ether leitet man unterhalb − 20 °C 100 g Chlorgas ein ; anschließend werden sämtliche flüchtigen Bestandteile der Reaktionsmischung bei 20 °C am Rotationsverdampfer abgezogen, der kristalline Rückstand in 1 000 ml Methylenchlorid aufgenommen und die erhaltene dunkelblaue Lösung bis zum Verschwinden der primär gebildeten Chlornitrosoverbindung bei Raumtemperatur aufbewahrt. Danach fügt man die nunmehr farblose Flüssigkeit binnen drei Stunden zu einer auf 10 bis 15 °C abgekühlten Suspension von 79,6 g Kaliumcyanid in 800 ml Methanol und läßt vier Stunden bei Raumtemperatur nachrühren. Das abgeschiedene Kaliumchlorid wird abgesaugt, das Filtrat eingeengt, der Rückstand in Essigester aufgenommen, dreimal mit Wasser gewaschen und über Magnesiumsulfat getrocknet. Nach dem Abziehen des Lösungsmittels, zuletzt bei 0,001 mbar und 40 °C, verbleiben 102,85 g α-Oximinotetrahydrofuran-3-yl)-acetonitril in Form eines viskosen Öles, welches nach kurzer Zeit zu einer kristallinen Masse erstarrt, die ab 28 °C schmilzt ; Ausbeute : 66 % der Theorie.

$C_6H_8N_2O_2$ (140)

Ber. : C 51,4 H 5,8 N 20,0

Gef. : C 51,5 H 5,8 N 19,7

100-MHz-H-NMR-Spektrum in $CDCl_3$ (δ-Werte) :

2,0-2,5 (2H), 3,1-3,5 (1H), 3,65-4,2 (4H).

7,0 g α-Oximino-(tetrahydrofuran-3-yl)-acetonitril und 9,43 g Thiophosphorsäure-O,O-diethylesterchlorid werden in 65 ml Aceton vorgelegt und unter intensivem Rühren binnen zwei Stunden mit 7,25 g fein pulverisiertem Kaliumcarbonat versetzt. Die Reaktionsmischung wird zwei Tage bei Raumtemperatur gerührt, dann wird von unlöslichen Bestandteilen abgesaugt und das Filtrat unter vermindertem Druck eingeengt. Der Rückstand wird in Ether aufgenommen, dreimal mit Wasser gewaschen, über Natriumsulfat getrocknet und vom Lösungsmittel befreit. Nach dem Abdestillieren bei 0,01 mbar und 50 °C verbleiben 11,8 g (O,O-Diethylthiophosphoryl)-α-oximino-(tetrahydrofuran-3-yl)-acetonitril als nahezu farbloses, viskoses Öl ; Ausbeute : 80 % der Theorie.

$C_{10}H_{17}N_2O_4PS$ (292)

Ber. : C 41,4 H 5,9 N 9,5

Gef. : C 41,5 H 6,0 N 9,2

60-MHz-H-NMR-Spektrum in $CDCl_3$ (δ-Werte) : 1,2-1,6 (6H), 2,0-2,5 (2H), 3,05-3,75 (1H), 3,8-4,6 (8H).

## Beispiel 2

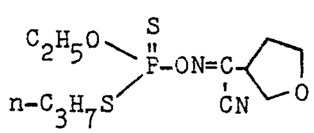

Eine Lösung von 7,0 g α-Oximino-(tetrahydrofuran-3-yl)-acetonitril in 40 ml absolutem Tetrahydrofuran wird auf − 40 °C abgekühlt und tropfenweise mit 31,2 ml einer 1,6 molaren Lösung von n-Butyllithium in n-Hexan versetzt. Die so erhaltene Mischung wird innerhalb von 30 Minuten auf − 20 °C erwärmt. Dann fügt man 10,92 g Dithiophosphorsäure-O-ethyl-S-n-propyl-diesterchlorid in 20 ml absolutem Tetrahydrofuran hinzu. Nach 24-stündiger Reaktionszeit bei Raumtemperatur wird das Lösungsmittel unter vermindertem Druck entfernt und der Rückstand mehrfach mit Ether extrahiert. Die vereinigten Extrakte werden dreimal mit Wasser gewaschen, über Natriumsulfat getrocknet und vom Lösungsmittel befreit. Man erhält 13,3 g (O-Ethyl-S-n-propyl-dithiophosphoryl)-α-oximino-(tetrahydrofuran-3-yl)-acetonitril als gelbliches Öl ; Ausbeute : 83 % der Theorie.

$C_{11}H_{19}N_2O_3PS_2$ (322)

Ber. : C 41,0 H 5,9 N 8,7

Gef. : C 41,4 H 6,3 N 8,6

220-MHz-H-NMR-Spektrum in $CDCl_3$ (δ-Werte) :

1,0 (3H), 1,35 (3H), 1,6-1,8 (2H), 2,0-2,5 (2H), 2,75-3,1 (3H), 3,5 (1H), 3,6-4,1 (4H), 4,2-4,35 (2H).

Beispiel 3

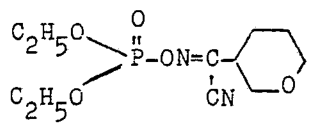

Analog Beispiel 1 erhält man durch Umsetzung von 114 g 3-Formyltetrahydropyran (Kp : 69 °C/18,6 mbar) mit 76,4 g Hydroxylaminohydrochlorid in Gegenwart von 44 g Natriumhydroxid nach Aufarbeitung und Destillation 78,6 3-Formyl-tetrahydropyranoxim als farblose, wasserklare Flüssigkeit : Kp : 85 bis 87 °C/0,13 mbar ; Ausbeute : 61 % der Theorie.

$C_6H_{11}NO_2$ (129)
Ber. : C 55,8 H 8,6 N 10,8
Gef. : C 56,0 H 8,4 N 11,0
60-MHz-H-NMR-Spektrum in $CDCl_3$ (δ-Werte) :
1,1-2,1 (4H), 2,1-2,7 (anti), 2,8-3,4 (syn) (1H), 3,0-4,1 (4H), 6,5 (syn), 7,2 (anti) (1H), 8,9-9,8 (1H).

Nach der im Beispiel 1 beschriebenen Arbeitsweise erhält man aus 67 g 3-Formyltetrahydropyran oxim, 40,5 g Chlor und 37,17 g Kaliumcyanid, 73,3 g α-Oximino-(tetrahydropyran-3-yl)-acetonitril mit einem Schmelzbereich von 46 bis 50 °C ; Ausbeute : 91 % der Theorie.

$C_7H_{10}N_2O_2$ (154)
Ber. : C 54,6 H 6,6
Gef. : C 54,9 H 6,8
220-MHz-H-NMR-Spektrum in $CDCl_3$ (δ-Werte) :
1,4-1,9 (3H), 1,9-2,2 (1H), 2,6-2,8 (1H), 3,3-3,8 (2H), 3,8-4,3 (2H), 10,6-11,4 (1H).

Aus 8,47 g α-Oximino-(tetrahydropyran-3-yl)-acetonitril, 11,36 g Kaliumcarbonat und 9,96 g Phosphorsäure-O,O-diethylesterchlorid lassen sich nach der in Beispiel 1 erläuterten Arbeitsvorschrift 13,44 g (O,O-Diethylphosphoryl)-α-oximino-(tetrahydropyran-3-yl)-acetonitril als nahezu farbloses, viskoses Öl gewinnen ; Ausbeute : 84 % der Theorie.

$C_{11}H_{19}N_2O_5P$ (290)
Ber. : C 45,5 H 6,6 N 9,6
Gef. : C 45,2 H 6,8 N 9,6
60-MHz-H-NMR-Spektrum in $CDCl_3$ (δ-Werte) :
1,3 (6H), 1,4-2,2 (4H), 2,5-3,0 (1H), 3,0-4,5 (6H).

Beispiel 4

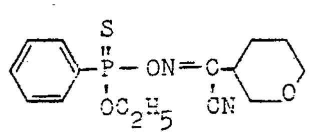

8,83 g Benzol-thiophosphonsäure-O-ethylesterchlorid läßt man zu einer Lösung von 6,16 g α-Oximino-(tetrahydropyran-3-yl)-acetonitril in 60 ml Acetonitril tropfen. Unter intensivem Rühren werden 8,24 g Kaliumcarbonat portionsweise hinzugefügt. Die Reaktionsmischung wird nach beendeter Zugabe noch 12 Stunden auf 40 °C gehalten. Zur Aufarbeitung wird das gebildete Kaliumchlorid durch Filtration entfernt ; das Filtrat eingeengt, der Rückstand in Essigester aufgenommen und gründlich mit Wasser gewaschen. Nach dem Trocknen über Natriumsulfat werden die Lösungsmittelreste, zuletzt bei 0,07 mbar und 30 °C, entfernt ; es verbleiben 12,4 g (O-Ethyl-benzolthiophosphonyl)-α-oximino-(tetrahydropyran-3-yl)-acetonitril ; Ausbeute : 92 % der Theorie.

$C_{15}H_{19}N_2O_3PS$ (338)
Ber. : C 53,3 H 5,7 N 8,2
Gef. : C 53,2 H 5,7 N 7,9
60 MHz-N-NMR-Spektrum in $CDCl_3$ (δ-Werte) :
1,45 (3H), 1,3-2,4, 2,5-3,1 (1H), 3,2-4,85 (6H), 7,5-7,9 (3H), 7,9-8,5 (2H).

Beispiel 5

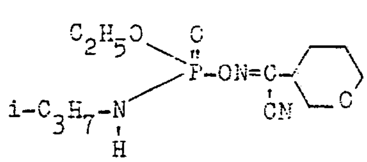

Analog Beispiel 4 erhält man durch Umsetzung von 7,7 g α-Oximino-(tetrahydropyran-3-yl)-acetonitril mit 9,27 g Phosphorsäure-ethylester-N-isopropylamino-chlorid in Gegenwart von 10,3 g Kaliumcarbonat 13,1 g (O-Ethyl-N-isopropylamidphosphoryl)-α-oximino-(tetrahydropyran-3-yl)-acetonitril.

$C_{12}H_{22}N_3O_4P$ (303)
Ber.: C 47,5 H 7,3 N 13,8
Gef.: C 47,7 H 7,4 N 13,5
80-MHz-H-NMR-Spektrum in $CDCl_3$ (δ-Werte):
1,1-1,3 (6H), 1,3-155 (3H), 1,6-2,3 (5H), 2,5-3,0 (1H), 3,1-4,0 (5H), 3,9-4,3 (2H).

Beispiel 6

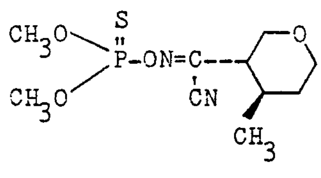

Nach der im Beispiel 1 erläuterten Arbeitsweise erhält man aus 76,4 g Hydroxylaminhydrochlorid, 44 g Natriumhydroxid und 128 g 4-Methyl-3-formyl-tetrahydropyran (Kp: 72 °C/17,3 mbar) 123 g des entsprechenden Aldoxims mit einem Siedebereich von 89 bis 95 °C bei 0,13 mbar; Ausbeute: 86 % der Theorie.

$C_7H_{13}NO_2$ (143)
Ber.: C 58,7 H 9,2 N 9,8
Gef.: C 58,5 H 9,0 N 10,1
60 MHz-H-NMR-Spektrum in $CDCl_3$ (δ-Werte):
0,8-1,1 (3H), 1,2-1,8 (3H), 1,9-2,5 (1H), 2,9-3,6 (2H), 3,7-4,2 (2H), 6,4 syn, 7,2 (anti) (1H), 8,7-9,9 (1H).

71,5 g 4-Methyl-3-formyltetrahydropyranoxim werden analog zu dem in Beispiel 1 erläuterten Synthesegang zunächst mit 39,05 g Chlor und anschließend mit 35,81 g Kaliumcyanid zur Reaktion gebracht. Man erhält 68,8 g α-Oximino-(4-methyl-tetrahydropyran-3-yl)-acetonitril mit einem Schmelzbereich von 94 bis 96 °C; Ausbeute: 77 % der Theorie.

$C_8H_{12}N_2O_2$ (169)
Ber.: C 56,9 H 7,2 N 16,6
Gef.: C 56,9 H 7,2 N 16,3
60-MHz-H-NMR-Spektrum in $CDCl_3$ (δ-Werte):
0,9-1,3 (3H), 1,3-2,2 (3H), 2,2-2,9 (1H), 3,3-3,9 (2H), 3,9-4,4 (2H), 10,0-10,9 (1H).

Durch Umsetzung von 8,45 g dieses Oxims mit 8,25 g Thiophosphorsäure-O,O-dimethylester-chlorid in Gegenwart von 10,32 g Kaliumcarbonat lassen sich 13,93 g (O,O-Dimethylthiophosphoryl)-α-oximino-(4-methyltetrahydropyran-3-yl)-acetonitril als gelbliches Öl gewinnen.

$C_{10}H_{17}N_2O_4PS$ (292)
Ber.: C 41,1 H 5,9 N 9,5
Gef.: C 41,2 H 6,1 N 9,2
60 MHz-H-NMR-Spektrum in $CDCl_3$ (δ-Werte):
1,0 (3H), 1,2-2,2 (3H), 2,25-2,7 (1H), 3,0-4,1 (4H), 3,7 (6H).

Beispiel 7

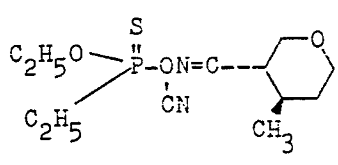

Analog Beispiel 6 werden 8,4 g α-Oximino-(4-methyltetrahydropyran-3-yl)-acetonitril mit 8,62 g Ethanthiophosphonsäure-O-ethylester-chlorid und 10,3 g Kaliumcarbonat zur Reaktion gebracht; man erhält 14,2 g (O-Ethylethanthiophosphonyl)-α-oximino-(4-methyltetrahydropyran-3-yl)-acetonitril; Ausbeute: 93 % der Theorie.

$C_{12}H_{21}N_2O_3PS$ (304)
Ber.: C 47,4 H 6,9 N 9,2
Gef.: C 47,7 H 7,1 N 9,3
80-MHz-H-NMR-Spektrum in $CDCl_3$ (δ-Werte):
0,9-1,5 (9H), 1,5-2,7 (7H), 3,2-4,4 (6H).

Die folgenden Verbindungen der Formel I können beispielsweise analog hergestellt werden:

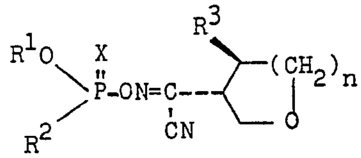

| Nr. | $R^1$ | $R^2$ | $R^3$ | X | n | H-NMR-Daten (MHz, Lösungsmittel, δ-Werte) |
|---|---|---|---|---|---|---|
| 8 | $CH_3$ | $CH_3O$ | $CH_3$ | O | 2 | (60, $CDCl_3$) 1,0 (3H), 1,2-2,2 (3H), 2,25-2,7 (1H), 3,05-4,1 (4H), 3,6 (6H) |
| 9 | $C_2H_5$ | $C_2H_5O$ | $CH_3$ | O | 2 | (100, $CDCl_3$) 1,0 (3H), 1,35 (6H), 1,4-2,15 (3H), 2-2,7 (1H), 3,2-3,6 (2H), 3,8-4,15 (2H), 4,05-4,4 (4H) |
| 10 | $C_2H_5$ | $C_2H_5O$ | $CH_3$ | S | 2 | (60, $CDCl_3$) 1,05 (3H), 1,35 (6H), 1,4-2,1 (3H), 2,3-2,8 (1H), 3,2-3,7 (2H), 3,75-4,6 (6H) |
| 11 | $C_2H_5$ | S—i—$C_4H_9$ | $CH_3$ | S | 2 | (60, $CDCl_3$), 0,8-2,2 (17H), 2,3-2,85 (1H). 3,1-3,7 (3H), 3,7-(4,6 (4H) |
| 12 | $C_2H_5$ | S—n—$C_3H_7$ | $CH_3$ | S | 2 | (60, $CDCl_3$) 0,8-1,2 (6H), 1,3-2,1 (5H), 1,4 (3H), 2,4-3,8 (5H), 3,8-4,6 (4H) |
| 13 | $C_2H_5$ | O—$C_2H_5$ | H | S | 2 | (220, $CDCl_3$) 1,3 (6H), 1,6-1,8 (3H), 2,0-2,2 (1H), 3,3-3,7 (2H), 3,9 (1H), 4,05 (1H) 4,1-4,4 (4H) |
| 14 | $CH_3$ | $CH_3O$ | H | O | 2 | (220, $CDCl_3$) 1,6-2,0 (3H), 2,05-2,3 (1H), 2,8-3,1 (1H), 3,4-3,7 (2H), 3,95 (6H), 4,0-4,2 (1H) |
| 15 | $C_2H_5$ | S—i—$C_4H_9$ | H | S | 2 | (60, $CDCl_3$) 0,9-1,2 (3H), 1,3-1,6 (6H), 1,5-2,3 (8H), 2,6-3,1 (1H), 3,2-4,6 (7H) |
| 16 | $CH_3$ | $OCH_3$ | H | S | 2 | (60, $CDCl_3$) 1,5-2,4 (4H), 2,5-3,2 (1H), 3,2-4,3 (4H), 3,9 (6H) |
| 17 | $C_2H_5$ | $CH_3$ | H | S | 2 | (60, $CDCl_3$) 1,3 (3H), 1,4-2,2 (4H), 2,95 (3H), 2,55-3,15 (1H), 3,1-4,1 (6H) |
| 18 | $C_2H_5$ | S—n—$C_3H_7$ | H | S | 2 | (60, $CDCl_3$) 1,0 (3H), 1,4 (3H), 1,5-2,2 (6H), 2,5-3,2 (3H), 3,2-4,5 (4H) |
| 19 | $C_2H_5$ | $C_6H_5$ | S | S | 2 | (60, $CDCl_3$) 0,95 (3H), 1,1-2,2 (6H), 2,3-2,85 (1H), 3,2-3,8 (2H), 3,85-4,3 (2H), 4,3-4,4,8 (2H), 7,4-8,5 (5H) |
| 20 | $C_2H_5$ | S—n—$C_3H_7$ | $CH_3$ | O | 2 | (60, $CDCl_3$) 0,9-1,3 (6H), 1,3-2,3 (5H), 1,45 (3H), 2,5-3,9 (5H), 3,9-4,7 (4H) |
| 21 | $C_2H_5$ | $NH_2$ | $CH_3$ | O | 2 | (80, $CDCl_3$) 1,0 (3H), 1,3-1,5 (3H), 1,5-2,2 (4H), 2,3-2,7 (1H), 3,1-3,6 (3H), 3,6-4,3 (4H) |
| 22 | $C_2H_5$ | S—n—$C_3H_7$ | H | O | 2 | (100, $CDCl_3$) 1,0 (3H), 1,35 (3H), 1,5-2,2 (6H), 2,6-3,1 (3H), 3,2-4,45 (6H) |
| 23 | $CH_3$ | $C_2H_5$ | $CH_3$ | S | 2 | (80, $CDCl_3$) 0,9-1,5 (6H), 1,5-19 (3H), 1,9-2,35 (2H), 2,35-2,7 (1H), 3,2-4,0 (4H), 3,7 (3H) |
| 24 | $CH_3$ | $C_2H_5$ | H | S | 2 | (100, $CDCl_3$) 1,0-1,5 (3H), 1,5-1,4 (4H), 1,9-2,4 (2H), 2,7-3,0 (1H), 3,3-4,2 (4H), 3,75 (3H) |
| 25 | $C_2H_5$ | $C_2H_5$ | H | S | 2 | (80, $CDCl_3$) 0,9-1,5 (6H), 1,5-2,0 (4H), 1,9-2,4 (2H), 2,6-2,95 (1H), 3,2-4,4 (6H) |
| 26 | $C_2H_5$ | NH—i—$C_3H_7$ | $CH_3$ | O | 2 | (80, $CDCl_3$) 0,9-1,15 (3H), 1,1-1,3 (6H), 1,3-1,5 (3H), 1,5-2,2 (4H), 2,3-2,7 (1H), 3,1-3,6 (2H), 3,6-4,3 (4H) |
| 27 | $C_2H_5$ | $N(CH_3)_2$ | $CH_3$ | O | 2 | (80, $CDCl_3$) 1,0 (3H), 1,35 (3H), 1,5-2,1 (3H), 2,3-2,8 (1H), 2,65 (6H), 3,1-3,6 (2H), 3,7-4,3 (4H) |
| 28 | $C_2H_5$ | $N(CH_3)_2$ | H | O | 2 | (80, $CDCl_3$) 1,2-1,5 (3H), 1,5-2,3 (4H), 2,5-3,0 (1H), 2,65 (6H), 3,2-3,85 (5H), 3,9-4,3 (2H) |

(Fortsetzung)

| Nr. | $R^1$ | $R^2$ | $R^3$ | X | n | H-NMR-Daten (MHz, Lösungsmittel, δ-Werte) |
|---|---|---|---|---|---|---|
| 29 | $C_2H_5$ | $C_2H_5$ | H | S | 1 | (220, $CDCl_3$) 1,05-1,55 (6H), 2,05-2,6 (4H), 3,4-3,6 (1H), 3,7-4,2 (4H), 4,2-4,45 (2H) |
| 30 | $CH_3$ | $OCH_3$ | H | S | 1 | (60, $CDCl_3$) 1,2-1,55 (3H), 1,8-2,65 (5H), 3,2-3,7 (1H), 3,7-4,8 (6H) |
| 31 | $C_2H_5$ | $CH_3$ | H | S | 1 | (60, $CDCl_3$) 1,2-1,55 (3H), 1,8-2,65 (5H), 3,2-3,7 (1H), 3,7-4,8 (6H) |
| 32 | $CH_3$ | $C_2H_5$ | H | S | 1 | (60, $CDCl_3$) 0,9-1,7 (3H), 1,9-2,65 (4H), 3,25-4,4 (8H) |
| 33 | $C_2H_5$ | S—i—$C_4H_9$ | H | S | 1 | (60, $CDCl_3$) 0,85-1,25 (3H), 0,85-2,5 (10H), 3,2-4,7 (8H) |
| 34 | $CH_3$ | $OCH_3$ | H | O | 1 | (80, $CDCl_3$) 2,0-2,55 (2H), 3,2-3,6 (1H), 3,7-4,6 (10H) |
| 35 | $C_2H_5$ | $OC_2H_5$ | H | O | 1 | (80, $CDCl_3$) 1,35 (6H), 2,0-2,5 (2H), 3,2-3,6 (1H), 3,7-4,45 (8H) |

Die erfindungsgemäßen Oximinophosphorsäurederivate der Formel I sind geeignet, Schädlinge aus der Klasse der Insekten, Milben, Zecken und Nematoden wirksam zu bekämpfen. Sie Können im Pflanzenschutz sowie auf dem Hygiene-, Vorratsschutz- und Veterinärsektor eingesetzt werden. Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler, Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling) ;

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner) ;

aus der Ordnung der Zweiflügler (Diptera) beispielsweise Lycoria pectoralis, Mayetiola destructor (Hessenfliege), Dasineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleas (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Ades aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ocis, Chrysomya macellaria, Chrysomya hominivorax, Lucilia cuprina, Lucilia sericata, Hypoderma lineata ;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise) ;

aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze) ;

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysaphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauekirschenlaus), Dysaulacorthum pseudosolani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus) ;

aus der Ordnung der Termiten (Isoptera) beispielsweise Reticulitermes lucifugus, Calotermes flavicollis, Leucotermes flavipes, Termes natalensis ;

aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrekke), Melanoplus spretus (Felsengebirgsheuschrecke), Melano plus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Zur Klasse der Arachnoidea gehören Milben und Zecken (Acarina) beispielsweise Ixodes ricinus (Holzbock), Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa.

Zur Klasse der Nemathelminthes zählen beispielsweise Wurzelgallennematoden, z.B. Meloidogyne incognita, Meloidogyne hapla, Meloidogyne javanica, Zysten bildende Nematoden, z.B. Heterodera rostochiensis, Heterodera schachtii, Heterodera avenae, Heterodera glycines, Heterodera triflolii, Stock- und Blattälchen, z.B. Ditylenchus dipsaci, Ditylenchus destructor, Pratylenchus neglectus, Pratylenchus penetrans, Partylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind :

I. 3 Gewichtsteile des Wirkstoffs Nr. 1 werden mit 97 Gewichtsteilen feinteiligem Kaolin innig vermischt. Man erhält auf diese Weise ein Stäubemittel, das 3 Gew.% des Wirkstoffs enthält.

II. 30 Gewichtsteile des Wirkstoffs Nr. 1 werden mit einer Mischung aus 92 Gewichtsteilen pulverförmigem Kieselsäuregel und 8 Gewichtsteilen Paraffinöl, das auf die Oberfläche diese Kieselsäuregels gesprüht wurde, innig vermischt. Man erhält auf diese Weise eine Aufbereitung des Wirkstoffs mit guter Haftfähigkeit.

III. 20 Gewichtsteile des Wirkstoffs Nr. 7 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoethanol-amid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 30 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden. Im allgemeinen liegen sie zwischen 0,000 1 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Zu den Wirkstoffen können Öle verschiedenen Typs, Herbizide, Fungizide, andere Insektizide, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden : 1,2-Dibrom-3-chlorpropan, 1,3-Dichlorpropen, 1,3-Dichlorpropen + 1,2-Dichlorpropan, 1,2-Dibrom-ethan, 2-sec.-Butylphenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethylbenzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N-[(methylcarbamoyl)-oxyl]-thio-acetimidat, Methyl-N',N'-dimethyl-N-[(methylcarbamoyl)oxyl]-1-thiooxamidat, N-(2-Methyl-4-chlor-phenyl)-N',N'-dimethylformamidin, Tetrachlorthiophen, 1-(2,6-Difluor-benzoyl)-3-(4-chlor-phenyl)-harnstoff, O,O-Dimethyl-O-(p-nitrophenyl)-phosphorthioat, O,O-Diethyl-O-(p-nitrophenyl)-phosphorthioat, O-Ethyl-O-(p-nitrophenyl)-phenyl-phosphonothioat, O,O-Dimethyl-O-(3-methyl-4-nitrophenyl)-phosphorthioat, O,O-Diethyl-O-(2,4-dichlorphenyl)-phosphorthioat, O-Ethyl-O-(2,4-dichlorphenyl)-phenyl-phosphonothioat, O,O-Dimethyl-O-(2,4,5-trichlorphenyl)- -phosphorthioat, O-Ethyl-O-(2,4,5-trichlorphenyl)-ethyl-phosphonothioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-[-p-methylsulfinyl)-phenyl]-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[-2-chlor-1-(2,4,5-trichlorphenyl)]-vinylphosphat, O,O-Dimethyl-S-(1′-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O,-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxyvinyl)-phosphat, O,O-Dimethyl-S-(N-methyl-carbamoylmethyl)-phosphorthioat, O,O-Dimethyl-S-(N-methyl-carbamoyl-methyl)-phosphorthioat, O,O-Dimethyl-S-(N-Methoxyethyl-carbamoyl-methyl)-phosphordithioat, O,O-Dimethyl-S-(N-formyl-N-methyl-carbamoylmethyl-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthio-methyl)-phosphordithioat, O,O-Diethyl-S-[(p-chlorphenylthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthioethyl)-phosphordithioat, O,O-Diethyl-S-(2-ethylsulfinylethyl)-phosphorthioat, O,O-Diethyl-thiophosphoryliminophenyl-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O[2-isopropyl-4-methyl-pyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amido-thioat, O,S-Dimethyl-N-acetyl-phosphoramidothioat, γ-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,-trans-chrysanthemat, 5-Benzyl-furyl-(3)-methyl-DL-cis,-trans-chrysanthemat, 3-Phenoxybenzyl(±)-cis,trans-2,2-dimethyl-3-(2,2-dichlorvinyl)-cyclopropancarboxylat, α-Cyano-3-phenoxybenzyl(±)-cis,trans-2,2-dimethyl-3-(2,2-dichlorvinyl)-cyclopropancarboxylat, (s)-α-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,trans-chrysanthemat, 2-Methyl-5-(2-propinyl)-3-furylmethyl-chrysanthemat, (α-Cyano-3-phenoxy-benzyl)-α-isopropyl-4-chlorphenylacetat.

Die folgenden Beispiele belegen die biologische Wirkung der neuen Verbindungen. Vergleichsmittel sind die bekannten Wirkstoffe O,O-Diethyl-S-(2-Chlor-1-phthalimidoethyl)-phosphordithioat (DE-AS 15 45 977), O,O-Diethyl-acetonoxim-thiophosphat (DE-AS 1 052 981), O,O-Dimethylthionophosphoryl-α-oximino-2-methylphenyl-essigsäurenitril (DE-AS 12 38 902) und O,O-Dimethyl-(1-cyan-1-cyclohexyl)-ketonoxim-thionophosphorsäureester (DE-OS 2 304 848).

Die Numerierung der Wirkstoffe entspricht der der Beispiele und der tabellarischen Auflistung.

## Beispiel A

Kontaktwirkung auf Malathion-resistente Reismehlkäfer (Tribolium castaneum).

Reismehlkäfer werden innerhalb von Glasringen mit einem Durchmesser von 4,5 cm auf mit acetonischer Wirkstofflösung behandelten Rundfiltern (Durchmesser 0,7 cm) exponiert.

Nach 24 Stunden wird die Mortalitätsrate ermittelt. Dabei zeigt sich, daß die Wirkstoffe Nr. 1, 6, 7, 8, 9, 10, 13, 14, 16, 17, 29, 30, 31 und 32 bekannten Wirkstoffen in ihrer Wirkung stark überlegen sind.

## Beispiel B

Kontaktwirkung auf Kornkäfer (Sitophilus granaria).

Petrischalen von 10 cm Durchmesser werden mit acetonischer Wirkstofflösung ausgekleidet. Nach dem Verdunsten des Lösungsmittels belegt man die Schalen mit 100 Kornkäfern. Nach 4 Stunden werden die Käfer in unbehandelte Gefäße überführt. Die Mortalitätsrate wird nach 24 Stunden ermittelt. Dabei wird festgestellt, wieviele Käfer in der Lage sind, nach diesem Zeitpunkt innerhalb 60 Minuten ein unbehandeltes Pappschälchen (Durchmesser 40 mm, Höhe 10 mm) zu verlassen.

Bei Kornkäfern, die mit den Wirkstoffen Nr. 3, 6, 7, 8, 9, 10, 13, 14, 16 und 17 behandelt wurden, ist die Mortalitätsrate bei vergleichbarer Wirkstoffmenge pro Petrischale wesentlich höher als bei Käfern, die mit bekannten Wirkstoffen behandelt wurden.

## Beispiel C

Kontaktwirkung auf Schaben (Blatta orientalis)

Der Boden eines 1 l-Einmachglases wird mit der acetonischen Lösung des Wirkstoffes behandelt.

Nach dem Verdunsten des Lösungsmittels setzt man je Glas 5 adulte Schaben. Die Mortalität wird nach 48 Stunden bestimmt.

In diesem Test zeigen die Wirkstoffe Nr. 3, 6, 7, 8, 9, 10, 13, 15, 16 und 17 eine bessere Wirkung als bekannte Wirkstoffe.

Beispiel D

Kontaktwirkung auf Stubenfliegen (Musca domestica) ; Applikationstest.

4-Tage alte Imagines erhalten in leichter $CO_2$-Narkose 1 µl der acetonischen Lösung des Wirkstoffes auf das ventrale Abdomen appliziert. Hierzu wird eine Mikrometerspritze verwendet.

Je 20 Versuchstiere mit gleicher Behandlung bringt man dann in einen Folienbeutel von ca. 500 ml Inhalt. Nach 4 Stunden zählt man die Tiere in Rückenlage aus und ermittelt graphisch die LD 50.

Die LD 50 der Wirkstoffe 3, 7, 8, 13, 16, 17 und 23 ist niedriger als die bekannter Wirkstoffe.

Beispiel E

Kontaktwirkung auf Blattläuse (Aphis fabae) ; Spritzversuch

Getopfte Bohnenpflanzen (Vicia faba) mit starken Blattlauskolonien werden in einer Spritzkammer mit wäßrigen Wirkstoffaufbereitungen tropfnaß gespritzt. Die Auswertung erfolgt nach 48 Stunden.

Es zeigt sich, daß — im Vergleich zu der erforderlichen Menge an bekannten Wirkstoffen — mit wesentlich niedrigeren Mengen der Wirkstoffe 3, 5, 6, 7, 11, 13, 14, 15, 16, 17, 23, 26 und 27 eine Mortalitätsrate von 100 % erzielt wird.

Beispiel F

Kontaktwirkung auf Baumwollwanzen (Dysdercus intermedius)

Petrischalen von 10 cm Durchmesser werden mit 1 ml acetonischer Wirkstofflösung ausgekleidet.

Nach dem Verdunsten des Lösungsmittels besetzt man die Schalen mit je 20 Larven des vorletzten Stadiums und registriert die Wirkung nach 24 Stunden.

In diesem Test zeigen die Wirkstoffe Nr. 3, 6, 8, 9, 10, 13, 14, 16, 17 und 22 eine bessere Wirkung als Vergleichsmittel.

Beispiel G

Kontaktwirkung auf Zecken (Ornithodorus moubata)

Geprüft wird mit Zecke im 3. Larvenstadium. Dazu taucht man die Tiere, die sich in einem Papierbeutel befinden, für 3 Sekunden in die Prüfemulsion. Die Beutel werden frei aufgehängt. Nach 48 Stunden wird die Wirkung auf die Zecken beurteilt.

In diesem Test wird mit Prüfemulsionen wesentlich geringerer Konzentration der Wirkstoffe 3, 5, 7, 9, 10, 13, 14, 17 eine Mortalitätsrate von 100 % erzielt als mit Prüfemulsion, die bekannte Wirkstoffe in höherer Konzentration enthalten.

Beispiel H

Wirkung auf Wurzelgallennematoden (Meloidogyne incognita)

Die Versuche werden mit je 500 g Komposterde durchgeführt, die stark mit Wurzelgallennematoden infiziert ist. Die Behandlung erfolgt mit der wäßrigen Wirkstoffaufbereitung durch Angießen in 20 ml der Aufbereitung.

Nach 6 bis 8 Wochen bonitiert man die Wurzeln auf Gallenbildung. Dabei zeigt sich, daß 0,1 %ige Aufbereitungen der Wirkstoffe 3, 10 und 11 in diesem Test die Gallenbildung verhindern.

**Ansprüche**

1. Oximinophosphorsäurederivate der Formel I

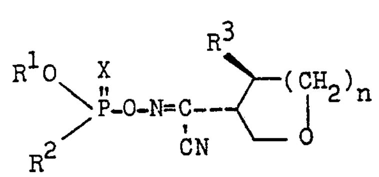

(I)

in der

$R^1$ eine unverzweigte oder verzweigte Alkylgruppe mit bis zu 4 Kohlenstoffatomen,

$R^2$ eine unverzweigte oder verzweigte Alkoxy- oder Alkylthiogruppe mit bis zu 4 Kohlenstoffatomen, eine unverzweigte oder verzweigte Alkylgruppe mit bis zu 3 Kohlenstoffatomen, Phenyl, die Aminogruppe oder einen unverzweigten oder verzweigten Alkylamino- oder Dialkylaminorest mit jeweils bis zu 4 Kohlenstoffatomen in einer Alkylgruppe,

$R^3$ Wasserstoff oder Methyl,

X Sauerstoff oder Schwefel und

n 1 oder 2 bedeuten.

2. (O,O-Diethylthiophosphoryl)-α-oximino-tetrahydropyran-3-yl-acetonitril.

3. (O-Ethylmethanthiophosphoryl)-α-oximino-tetrahydropyran-3-yl-acetonitril.

4. Verfahren zur Herstellung von Oximinophosphorsäurederivaten der Formel I gemäß Anspruch 1, dadurch gekennzeichnet, daß man α-Oximinonitrile der Formel II

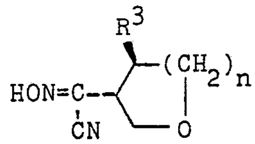

(II)

in der $R^3$ und n die im Anspruch 1 genannten Bedeutungen haben, gegebenenfalls in Gegenwart eines Säureacceptors oder Alkalimetall-, Erdalkalimetall- oder gegebenenfalls substituierte Ammoniumsalze dieser α-Oximinonitrile mit (Thio) (Thiol)Phosphor(Phosphon)säureester(amid)halogeniden der Formel III

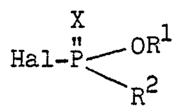

(III)

in der $R^1$, $R^2$ und X die im Anspruch 1 genannten Bedeutungen haben und Hal für Halogen steht, gegebenenfalls in Gegenwart eines Lösungs- oder Verdünnungsmittels bei Temperaturen zwischen 0 und 120 °C umsetzt.

5. Schädlingsbekämpfungsmittel, enthaltend ein Oximinophosphorsäurechlorid der Formel I gemäß Anspruch 1.

6. Schädlingsbekämpfungsmittel, enthaltend feste und/oder flüssige Zusatzstoffe und ein Oximinophosphorsäurederivat der Formel I gemäß Anspruch 1.

7. Verfahren zur Bekämpfung von Schädlingen, dadurch gekennzeichnet, daß man Oximinophosphorsäurederivate der Formel I gemäß Anspruch 1 auf Schädlinge bzw. deren Lebensraum einwirken läßt.

**Claims**

1. An oximinophosphoric acid derivative of the formula I

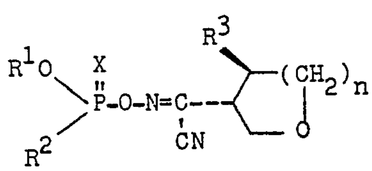

(I)

where

$R^1$ is unbranched or branched alkyl of up to 4 carbon atoms,

$R^2$ is unbranched or branched alkoxy or alkylthio of up to 4 carbon atoms, unbranched or branched alkyl of up to 3 carbon atoms, phenyl, amino, alkylamino or dialkylamino, where each alkyl is unbranched or branched and of up to 4 carbon atoms,

$R^3$ is hydrogen or methyl,

X is oxygen or sulfur and

n is 1 or 2.

2. (O,O-Diethylthiophosphoryl)-α-oximino-tetrahydropyran-3-yl-acetonitrile.

3. (O,O-Ethylmethanethiophosphoryl)-α-oximino-tetrahydropyran-3-yl-acetonitrile.

4. A process for the manufacture of oximinophosphoric acid derivatives of the formula I as claimed in claim 1, wherein an α-oximinonitrile of the formula II

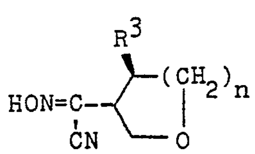

(II)

where $R^3$ and n have the meanings given in claim 1, is reacted in the presence or absence of an acid acceptor, or an alkali metal salt, alkaline earth metal salt or unsubstituted or substituted ammonium salt of such an α-oximinonitrile is reacted with a (thio) (thiol)-phosphoric(phosphonic) acid ester(amide) halide of the formula III

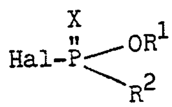

(III)

where $R^1$, $R^2$ and X have the meanings given in claim 1 and Hal denotes halogen, in the presence or absence of a solvent or diluent and at from 0° to 120 °C.

5. A pesticide containing an oximinophosphoric acid derivative of the formula I as claimed in claim 1.

6. A pesticide containing solid and/or liquid additives and an oximinophosphoric acid derivative of the formula I as claimed in claim 1.

7. A process for combating pests, wherein an oximinophosphoric acid derivative of the formula I as claimed in claim 1 is allowed to act on pests or their habitat.

**Revendications**

1. Dérivés d'acide oximinophosphorique de formule

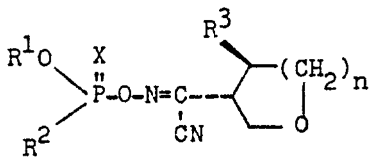

dans laquelle

$R^1$ représente un groupe alkyle ramifié ou non ramifié, ayant jusqu'à 4 atomes de carbone.

$R^2$ un groupe alcoxy- ou alkylthio ramifié ou non ramifié, ayant jusqu'à 4 atomes de carbone, un groupe alkyle ramifié ou non ramifié ayant jusqu'à 3 atomes de carbone, phényle, le groupe amino ou un reste alkylamino ou dialkylamino ramifié ou non ramifié ayant chacun jusqu'à 4 atomes de carbone dans un groupe alkyle,

$R^3$ hydrogène ou méthyle

X oxygène ou soufre et

n 1 ou 2.

2. (O,O-diéthylthiophosphoryl)-α-oximino-tétrahydropyran-3-yl-acétonitrile.

3. (O-éthylméthanthiophosphoryl)-α-oximino-tétrahydropyran-3-yl-acétonitrile.

4. Procédé de préparation de dérivés d'acide oximinophosphorique de formule 1 selon la revendication 1, caractérisé par le fait qu'on fait réagir, à des températures comprises entre 0 et 120 °C, éventuellement en présence d'un solvant ou diluant, un α-oximinonitrile de formule II

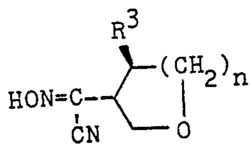

dans laquelle $R^3$ et n ont les significations indiquées dans la revendication 1, éventuellement en présence d'un accepteur d'acide ou de sels de métaux alcalins, de métaux alcalinoterreux ou de sels d'ammonium éventuellement substitués de cet α-oximinonitrile, avec des halogénures d'ester (amide) d'acide (thio) (thiol) phosphorique (phosphonique) de formule III

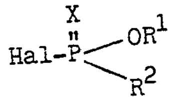

(III)

dans laquelle $R^1$, $R^2$ et X ont les significations données dans la revendication 1 et Hal représente un halogène.

5. Pesticide contenant un chlorure d'acide oximinophosphorique de formule I selon la revendication 1.

6. Pesticide contenant un additif solide et/ou liquide et un dérivé d'acide oximinophosphorique de formule I selon la revendication 1.

7. Procédé de lutte contre les parasites, caractérisé par le fait qu'on fait agir un dérivé d'acide oximinophosphorique de formule I selon la revendication 1 sur des parasites ou leur biotope.